Europäisches Patentamt

European Patent Office

Office européen des brevets

(19)

(11) Veröffentlichungsnummer : **0 147 823**
**B1**

(12) **EUROPÄISCHE PATENTSCHRIFT**

(45) Veröffentlichungstag der Patentschrift :
25.05.88

(51) Int. Cl.⁴ : **C 07 C 31/18, B 01 J  2/30**

(21) Anmeldenummer : **84116070.8**

(22) Anmeldetag : **21.12.84**

(54) **Verfahren zur Verbesserung der Rieselfähigkeit von Polyolen.**

(30) Priorität : **29.12.83 DE 3347405**

(43) Veröffentlichungstag der Anmeldung :
**10.07.85 Patentblatt 85/28**

(45) Bekanntmachung des Hinweises auf die Patenterteilung : **25.05.88 Patentblatt 88/21**

(84) Benannte Vertragsstaaten :
**AT DE FR GB IT NL SE**

(56) Entgegenhaltungen :
**EP-A- 0 047 556**
**US-A- 3 374 098**
**US-A- 4 304 798**
**CHEMICAL ABSTRACTS, Band 83, Nr. 2, 14. Juli 1975, Seite 17, Spalte 2, Zusammenfassungsnr. 11126k, Columbus, Ohio, US**

(73) Patentinhaber : **Ruhrchemie Aktiengesellschaft
Bruchstrasse 219
D-4200 Oberhausen 11 (DE)**

(72) Erfinder : **Bach, H., Dr., Dipl.-Chem.
Alleestrasse 56
D-4100 Duisburg 11 (DE)**
Erfinder : **Bötel, Heinz
Hugo-Rasch-Strasse 14
D-4200 Oberhausen (DE)**
Erfinder : **Hahn, H.-D., Dr., Dipl.-Chem.
Burgstrasse 21
D-4200 Oberhausen 11 (DE)**
Erfinder : **Thönnessen, F., Dr.,Dipl.-Chem.
Lützowstrasse 49
D-4200 Oberhausen 11 (DE)**

(74) Vertreter : **Reichelt, Karl-Heinz, Dr.
Ruhrchemie Aktiengesellschaft Abt. PLD Postfach 13 01 60
D-4200 Oberhausen 11 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 147 823 B1

## Beschreibung

Die Erfindung betrifft in neues Verfahren zur Verbesserung der Rieselfähigkeit durch Verhinderung des Verbackens fester Polyalkohole (Polyole) und führt damit zur Verbesserung ihrer Lagerfähigkeit unter Normalbedingungen.

Polyole sind wichtige Zwischenprodukte für chemische Synthesen, die teilweise großtechnische Bedeutung erlang haben und u. a. zur Herstellung von Kunststoffen, wie Polyurethanen, Polycarbonaten, Alkydharzen, Schmiermitteln, Weichmachern und Lacken, Verwendung finden. Maßgebend für ihren großen Anwendungsbereich ist die meist leichte Zugänglichkeit der Polyole.

Beispiele für technisch eingesetzte Polyole sind 2,2-Dimethyl-1,3-propandiol (Neopentylglykol), 1,6-Hexandiol und Trimethylolpropan. Ihre Herstellung erfolgt durch katalytische Hydrierung der entsprechenden Carbonsäuren oder Ester, wie im Fall der 1,6-Hexandiols, oder Mischaldolisierung von Aldehyden mit Formaldehyd und anschliessende Reduktion der Hydroxyaldehyde, wie beim 2,2-Dimethyl-1,3-propandiol oder dem Trimethylolpropan.

Um eine einfache Handhabung bei der Weiterverarbeitung sicherzustellen, werden die Polyole konfektioniert und kommen z. B. in Form von Schuppen, Pastillen oder Briketts in den Handel.

Als sehr störend bei der Lagerung erweist sich die Verbackungsneigung der Polyole. Die Ursache hierfür ist nicht immer bekannt. Beim 2,2-Dimethyl-1,3-propandiol wurde gefunden, daß es in festem Zustand in zwei unterschiedlichen Modifikationen, die reversibel ineinander überführbar sind, vorliegt. Die Umwandlungstemperaturen der thermodynamisch instabilen Modifikation in die thermodynamisch stabile Form und umgekehrt betragen 42 bzw. 33 °C bei Unterkühlung. Die Umwandlungswärme der beiden Modifikationen wurde zu 13,7 kJ/Mol ermittelt (H.P. Frank, K. Krzenicki, H. Völlenkle, Chemiker-Ztg. 97, 206 (1973)) und ist damit annähernd dreimal so groß wie die Schmelzwärme. Aufgrund dieser physikalischen Eigenschaften des 2,2-Dimethyl-1,3-propandiols verläuft die Verschuppung des Reinproduktes nicht unproblematisch. Schwierigkeiten treten — insbesondere bei hochreinen Qualitäten — dadurch auf, daß das zu konfektionierende Material z: B. auf der Schuppenwalze nicht vollständig in die thermodynamisch stabilere « Tieftemperatur-Modifikation » übergeht. Als Folge hiervon wandelt sich das Neopentylglykol später in den Verkaufsgebinden in die entsprechende « Tieftemperatur-Modifikation » um, wobei die sehr hohe Umwandlungswärme freigesetzt wird. Die abzuführende Umwandlungswärme bedingt einen beträchtlichen Temperaturanstieg in der Verkaufsware und führt zu mehr oder weniger ausgeprägten Verbackungen der verschuppten Ware. Im Extremfall wird die Rieselfähigkeit der Ware erheblich beeinträchtigt.

Zur Vermeidung der aufgezeigten Komplikationen wird in der japanischen Patentschrift 74 88 813 (CA 83, 11126 K (1975)) der Zusatz von Estern organischer Säuren oder von Acetalen als Antibackmittel beschrieben. Danach bewirkt der Zusatz von 0,0005 Gew.-% Celluloseacetat-Butylester, daß Neopentylglykol, das nach Konfektionierung über 30 Tage lang einem Druck von 230 p/cm$^2$ ausgesetzt worden war, keine Verbackungen zeigte.

Ein weiteres Verfahren zur Verbesserung der Rieselfähigkeit ist in der DE 30 10 138 C2 beschrieben. Hierbei setzt man den Polyolen tertiäre Amine, die mindestens 2 gleiche organische Substituenten besitzen, in einer Konzentration von 0,005 bis 0,25 Gew.-%, bezogen auf Polyol, zu.

Der Nachteil dieses Verfahrens besteht darin, daß die verwendeten Amine stets einen Eigengeruch aufweisen, der als störend empfunden wird. Darüberhinaus können die Aminanteile bei einer Weiterverarbeitung unerwünschte katalytische Wirkungen auslösen.

Da die beschriebenen Zusätze bei Erreichen höherer Drücke, wie sie bei der Lagerung in den heute üblichen Sackstapeln größerer Höhe auftreten, ihre Wirkung weitgehend verlieren und darüber hinaus bei der Weiterverarbeitung, z. B. auf Alkydharze, Polymere, Schmiermittel und Additive, Störungen hervorrufen, ergab sich somit die Aufgabe, die geschilderten Nachteile zu vermeiden und Antibackmittel, die auch unter ungünstigen Bedingungen wirksam sind, keinen störenden Eigengeruch aufweisen und bei einer Weiterverarbeitung keine unerwünschten katalytischen Wirkungen entfalten, zu finden.

Die Erfindung besteht in einem Verfahren zur Verbesserung der Rieselfähigkeit von unter Normalbedingungen kristallinen Polyolen, dadurch gekennzeichnet, daß man dem zu konfektionierenden Polyol vor Reindestillation oder Konfektionierung ein weiteres Polyol mit 5 bis 12 C-Atomen in einer Konzentration von 0,005 bis 0,4 Gew.-% bezogen auf Polyol, zusetzt. Als besonders geeignet haben sich aliphatische Diole mit 5 bis 12 C-Atomen erwiesen. Diese Diole können geradkettige oder auch substituierte aliphatische Diole, insbesondere 1,3-Diole, sein.

Bei den verwendeten 1,3-Diolen handelt es sich vorzugsweise um solche, die in 2-Stellung doppelt substituiert sind. Diese Substituenten stellen übliche aliphatische Reste, wie den Methyl-, Ethyl, n-Propyl, n-Butyl-, sek.-Butylrest, dar, sie können gleich oder verschieden sein.

Die zugesetzten Polyole werden entweder in reiner Form oder in Mischungen mit anderen Polyolen in einer Konzentration von 0,005 bis 0,4 Gew.-%, bezogen auf zu konfektionierendes Polyol, vorzugsweise in einer Konzentration von 0,02 bis 0,250 Gew.-%, zugesetzt.

Versuchsbeschreibung

Schmelzflüssiges Polyol (Diol) wird in einen beheizbaren Rührbehälter (Inhalt 600 l) eingefüllt und

2

das Antibackmittel über eine entsprechend dimensionierte Dosiervorrichtung, die ebenfalls beheizbar ist, zugesetzt. Unter Rühren wird das schmelzflüssige Polyol homogenisiert. Alternativ hierzu kann in der Dosiervorrichtung unter Rühren ein Konzentrat aus schmelzflüssigem Polyol und der für die Gesamtmenge an Polyol notwendigen Menge Antibackmittel angesetzt und dem Rührbehälter zugeführt werden.

Im Anschluß hieran wird das mit dem Antibackmittel versetzte, schmelzflüssige Polyol über eine gekühlte Schuppenwalze (Stundenleistung : 20 kg/h) geschickt. Die hierbei anfallenden Schuppen werden in Lagerversuchen auf ihre Eignung hin untersucht.

Die Tabelle gibt einige Versuchsbeispiele wieder. In allen beschriebenen Fällen zeigten die so hergestellten Schuppen keinerlei Verbackungseigenschaften, während die in Vergleichsversuchen ohne Zusatz von Antibackmitteln hergestellten Schuppen nach kurzer Lagerung infolge von Anbackungen und Verbackungen nicht mehr einwandfrei rieselfähig waren.

Die Versuchsergebnisse sind den nachfolgenden Tabellen zu entnehmen.

(Siehe Tabellen Seite 4 ff.)

Tabelle 1

| Zu konfektionie- rendes Polyol | Antibackmittel | Konzentration (Gew.-%) | Rieselfähigkeit nach | |
|---|---|---|---|---|
| | | | 1 Monat | 2 Monaten |
| 2,2-Dimethyl- propandiol-1,3 | Hexandiol-1,6 | 0,025 | gut | gut |
| 2,2-Dimethyl- propandiol-1,3 | 2-Methyl-2-n-propyl- propandiol-1,3 | 0,1 | sehr gut | gut |
| 2,2-Dimethyl- propandiol-1,3 | 2-Methyl-2-n-butyl- propandiol-1,3 | 0,05 | gut | gut |
| 2,2-Dimethyl- propandiol-1,3 | 2-Methyl-2-sek.-butyl- propandiol-1,3 | 0,02 | sehr gut | gut |
| 2,2-Dimethyl- propandiol-1,3 | 2-Ethyl-2-n-butyl- propandiol-1,3 | 0,15 | gut | gut |
| 2,2-Dimethyl- propandiol-1,3 | 2,2,4-Trimethyl-1,3- pentandiol | 0,2 | gut | gut |

0 147 823

Tabelle 2

| Zu konfektionierendes Polyol | Antibackmittel | Konzentration (Gew.-%) | Rieselfähigkeit nach | |
|---|---|---|---|---|
| | | | 1 Monat | 2 Monaten |
| 2-Methyl-2-sek.-butylpropandiol-1,3 | 2,2-Dimethyl-propandiol-1,3 | 0,1 | gut | gut |
| 2,2,4-Trimethyl-1,3-pentandiol | 2-Methyl-2-propyl-propandiol-1,3 | 0,2 | sehr gut | gut |
| 2-Ethyl-2-n-butyl-propandiol-1,3 | Hexandiol-1,6 | 0,05 | gut | gut |
| 2-Ethyl-2-n-butyl-propandiol-1,3 | 2-Methyl-2-sek.-butyl-propandiol-1,3 | 0,05 | sehr gut | gut |

## Patentansprüche

1. Verfahren zur Verbesserung der Rieselfähigkeit von unter Normalbedingungen kristallinen Polyolen, dadurch gekennzeichnet, daß man dem zu konfektionierenden Polyol vor Reindestillation oder Konfektionierung ein weiteres Polyol mit 5 bis 12 C-Atomen in einer Konzentration von 0,005 bis 0,4 Gew.-%, bezogen auf zu konfektionierendes Polyol, zusetzt.

2. Verfahren nach Anspruch 1, dadurch gekennzeichnet, daß man das weitere Polyol in einer Konzentration von 0,02 bis 0,25 Gew.-%, bezogen auf zu konfektionierendes Polyol, zusetzt.

3. Verfahren nach Anspruch 1 und 2, dadurch gekennzeichnet, daß das weitere, zugesetzte Polyol ein Diol mit 5 bis 12 C-Atomen ist.

4. Verfahren nach Anspruch 1 bis 3, dadurch gekennzeichnet, daß das weitere, zugesetzte Polyol ein aliphatisches Diol, insbesondere ein aliphatisches 1,3-Diol ist.

5. Verfahren nach Anspruch 4, dadurch gekennzeichnet, daß das aliphatische 1,3-Diol in 2-Stellung substituiert ist und der Substituent einen aliphatischen Rest aus der Reihe der Methyl-, Ethyl-, Propyl, n-Butyl-, und sek.-Butylgruppen darstellt.

6. Verfahren nach Anspruch 5, dadurch gekennzeichnet, daß das aliphatische 1,3-Diol in 2-Stellung doppelt substituiert ist und die Substituenten in der 2-Stellung gleich oder verschieden sind.

## Claims

1. A process to improve the free flowing properties of polyols which are crystalline under normal conditions, characterised in that another polyol with 5 to 12 carbon atoms in a concentration of 0.005 to 0.4 wt. %, related to the polyol to be fabricated, is added to the polyol to be fabricated before fine distillation or fabrication.

2. A process according to claim 1, characterised in that the other polyol is added in a concentration of 0.02 to 0.25 wt. %, related to the polyol to be fabricated.

3. A process according to claim 1 and 2, characterised in that the polyol added is a diol with 5 to 12 carbon atoms.

4. A process according to claims 1 to 3, characterised in that the polyol added is an aliphatic diol, in particular an aliphatic 1,3-diol.

5. A process according to claim 4, characterised in that the aliphatic 1,3-diol is substituted in position No. 2 and the substituent is an aliphatic radical from the methyl, ethyl, propyl, n-butyl and secondary butyl groups.

6. A process according to claim 5, characterised in that the aliphatic 1,3-diol is disubstituted in position No. 2 and the substituents in position No. 2 are the same or different.

## Revendications

1. Procédé pour l'amélioration de la faculté d'écoulement de polyols cristallisés en conditions normales, caractérisé en ce que l'on ajoute au polyol à confectionner, avant la purification par distillation ou la confection, un autre polyol en $C_5$-$C_{12}$ à une concentration de 0,005 à 0,4 % en poids, par rapport au polyol à confectionner.

2. Procédé selon la revendication 1, caractérisé en ce que l'on ajoute l'autre polyol à une concentration de 0,02 à 0,25 % en poids par rapport au polyol à confectionner.

3. Procédé selon les revendications 1 et 2, caractérisé en ce que l'autre polyol ajouté est un diol en $C_5$-$C_{12}$.

4. Procédé selon les revendications 1 à 3, caractérisé en ce que l'autre polyol ajouté est un diol aliphatique, en particulier en 1,3-diol aliphatique.

5. Procédé selon la revendication 4, caractérisé en ce que le 1,3-diol aliphatique est substitué en position 2 et le substituant est un reste aliphatique choisi parmi les groupes méthyle, éthyle, propyle, n-butyle et sec-butyle.

6. Procédé selon la revendication 5, caractérisé en ce que le 1,3-diol aliphatique est doublement substitué en position 2 et les substituants en position 2 sont identiques ou différents.